# EUROPÄISCHE PATENTANMELDUNG

(11) **EP 3 819 908 A1**
(43) Veröffentlichungstag der Anmeldung: **12.05.2021**
(21) Anmeldenummer: 20205123.1
(22) Anmeldetag: 02.11.2020
(51) Int. Cl.: G16H 20/40, G16H 20/30

(54) **VERFAHREN ZUR ERFASSUNG, ANALYSE UND OPTIMIERUNG VON BEWEGUNGSAUSFÜHRUNGEN IM SPORTBEREICH UND REHA-BEREICH**

(30) Priorität: 06.11.2019 DE 102019129846
(71) Anmelder: ERGO-FIT GmbH & Co. KG, 66955 Pirmasens (DE)
(72) Erfinder: RAUBER, Matthias, 66955 Pirmasens (DE); WEBER, Martin, 66969 Lemberg (DE)
(74) Vertreter: Patentanwälte Dr. Keller, Schwertfeger Partnerschaft mbB

(57) **Zusammenfassung**

Die vorliegende Erfindung betrifft ein System sowie ein Verfahren zur Erfassung, Analyse und Optimierung von Bewegungsausführungen im Sportbereich und Rehabereich. Das Verfahren umfasst die folgenden Schritte:
- Identifizieren einer Person durch Abgleich mit wenigstens einem in einer Datenbank hinterlegten Foto und/oder wenigsten einem biometrischen Merkmal mittels einer auf die Datenbank zugreifenden Gesichtserkennungseinrichtung und/oder einer Erkennungseinrichtung für biometrische Merkmale einer portablen Vorrichtung;
- Abrufen von Gesundheitsdaten und/oder Trainingsdaten zur identifizierten Person aus der Datenbank;
- Erfassen wenigstens einer Bewegungsausführung der identifizierten Person;
- Zuordnen und Durchführen einer Vergleichsanalyse der erfassten Bewegungsausführung der identifizierten Person mit wenigstens einem in der Datenbank hinterlegten Bewegungsmodell für Personen mit entsprechenden Gesundheitsdaten und/oder Trainingsdaten;
- Generieren optimierter Trainingsparameter aus der durchgeführten Vergleichsanalyse und
- Verwenden wenigstens eines optimierten Trainingsparameters zur Optimierung der Bewegungsausführung.

## Beschreibung

Die vorliegende Erfindung betrifft ein Verfahren zur Erfassung, Analyse und Optimierung von Bewegungsausführungen im Sport- und Reha-Bereich sowie ein entsprechendes System.

Fitnesssport dient in erster Linie der Verbesserung und Erhaltung der Gesundheit. Viele Menschen besuchen daher ein Fitnessstudio. Dort werden neben dem klassischen Training an Fitnessgeräten regelmäßig Sportarten wie beispielsweise Tanzen, Rückentraining oder Bodengymnastik angeboten. Aber auch Reha-Sportkurse werden dort durchgeführt.

Unabhängig davon, ob an Fitnessgeräten trainiert oder eine Sportart betrieben wird, kann langfristig eine Verbesserung und Erhaltung der Gesundheit nur erreicht werden, wenn die Übungen korrekt und sauber ausgeführt werden. Andernfalls drohen Überlastungen der Gelenke, was langfristig zu Gelenksschädigungen führen kann.

Werden Übungen in Kursen mit mehreren Teilnehmern unsauber oder falsch ausgeführt, so korrigiert üblicherweise ein Trainer die Person, welche die Übung falsch ausführt. Allerdings unterbricht dies den Trainingsablauf und kann störend für andere Teilnehmer eines Trainingskurses sein. Daher wird regelmäßig von Kurstrainern nur dann eingegriffen, wenn die Ausführung der Übung stark fehlerhaft ist. Jedoch können bei Kursen mit vielen Teilnehmern auch solche Fehler vom Kurstrainer übersehen werden, was im schlimmsten Fall zu Verletzungen führen kann.

Nicht viel besser sieht es grundsätzlich bei dem Training mit Fitnessgeräten aus. Sensoren - wie zum Beispiel ein Seilzugsensor - liefern zwar Leistungsdaten an die trainierende Person, aber keinerlei Informationen über die Qualität der Bewegungsausführung. Hier ist wieder der Fitnesstrainer gefragt, der allerdings nicht unbedingt für jedes Gerät qualifiziert ausgebildet ist und zudem nicht immer präsent ist, so dass falsche Bewegungsausführungen häufig über längere Zeit unbemerkt bleiben, was zu den oben beschriebenen Nachteilen führen kann.

Demnach ist es erforderlich, dass die teilnehmende Person für das Training professionell angeleitet wird und die Qualität der Ausführung der Übungen engmaschig kontrolliert bzw. überwacht wird.

Die WO 2018/140 653 A1 betrifft ein Fitness-Abonnementprogramm-System umfassend einen Server, der unter anderem eine Reihe von mikroprozessorausführbaren Trainingsvideos speichert. Mittels einer tragbaren Vorrichtung können visuelle und/oder akustische Informationen des mikroprozessorausführbaren Trainingsvideos mittels drahtloser Übertragung wiedergegeben werden. Hierbei kann das System Daten in Bezug auf den Teilnehmer aufzeichnen, der eine Übungsroutine eines Trainingsvideos nachahmt. Diese Daten können von einem Trainer ausgewertet werden. Zum Anzeigen, Übertragen und Aufzeichnen aller mikroprozessorausführbaren Daten kann eine Virtual-Reality-Ausrüstung genutzt werden.

Nachteilig bei diesem System ist es, dass letztlich nur vorgegebene Übungen nachgeahmt werden und eine automatisierte Überwachung der Qualität der Übungsausführung nicht vorgesehen ist. Außerdem kann das System individuelle Bedürfnisse einzelner Personen nicht berücksichtigen, falls hierfür kein entsprechendes Trainingsvideo auf dem Server zur Verfügung steht.

Es ist daher Aufgabe der vorliegenden Erfindung, ein Verfahren zur Erfassung, Analyse und Optimierung von Bewegungsausführungen und ein entsprechendes System bereitzustellen, mit dem Bewegungsausführungen im Sportbereich und Reha-Bereich sowohl im Zusammenhang mit Fitnessgeräten als auch bei freien Bewegungsausführungen optimiert werden können.

Diese Aufgabe wird gelöst durch ein Verfahren gemäß Anspruch 1 und ein System gemäß Anspruch 10. Bevorzugte Ausführungsformen finden sich in den Unteransprüchen wieder.

Das erfindungsgemäße Verfahren und das System ermöglichen sowohl die Feststellung einer suboptimalen bzw. fehlerhaften Bewegungsausführung als auch die Generierung von Optimierungsmöglichkeiten, um die Qualität der Bewegungsausführung und somit die Qualität und Effektivität der Übungseinheit zu steigern und so langfristig die Gesundheit des Trainierenden zu erhalten oder zu verbessern. Das Besondere dabei ist, dass das Verfahren und das System sowohl eingesetzt werden können, um beispielsweise die Arbeit eines Trainers zu erleichtern bzw. ihm die Möglichkeit zu geben, treffsichere Analysen der Bewegungsabläufe eines Trainierenden durchzuführen, selbst wenn der Trainer für diese Art von Training nicht ausgebildet ist. Ebenso ermöglicht es dem Trainierenden eine Selbstkontrolle, ob er die Bewegung korrekt ausführt. Außerdem sind das Verfahren und das System dazu ausgelegt, von dem Training an Geräten bis hin zu dem Ganzkörpertraining auf der Matte eingesetzt zu werden.

Hierzu sieht das erfindungsgemäße Verfahren als ersten Schritt eine automatisierte Identifizierung der Trainingsperson vor. Mittels einer Gesichtserkennungseinrichtung bzw. einer Erkennungseinrichtung für biometrische Merkmale wird ein Abgleich mit wenigstens einem in einer Datenbank hinterlegten Lichtbild (Foto) und/oder wenigstens einem alternativen oder zusätzlichen biometrischen Merkmal durchgeführt. Dabei kann beispielsweise die zu identifizierende Person eine portable Vorrichtung, z.B. eine Digital-Reality (DR) oder Virtual-Reality (VR) Brille tragen und beispielsweise über einen Iris-Scan identifiziert werden. Es ist aber auch möglich, dass insbesondere ein Trainer die portable Vorrichtung trägt und die zu identifizierende Person beispielsweise über ihre Körperstatur, Proportionen, ihre Größe oder über sichtbare Merkmale ihres Gesichts identifiziert wird.

Nach einer erfolgreichen Identifikation der Person werden personenspezifische Gesundheitsdaten und/oder Trainingsdaten aus einer Datenbank abgerufen.

Unter Gesundheitsdaten sind alle Daten zu verstehen, die den Gesundheitszustand des Trainierenden betreffen, also solche Daten, die häufig auch in einer Trainingsakte oder Patientenakte zu finden sind. Dazu gehören unter anderem die Krankengeschichte (Anamnese), Informationen über aktuelle Erkrankungen, Diagnosen, Therapien (auch Eingriffe) und deren Verlauf, Informationen zu chronischen Erkrankungen, Vorerkrankungen, andere gesundheitsbezogene Informationen, wie z.B. Gewicht, Körperfettwerte, Blutzuckerwerte, Ernährungstagebuch, Medikamentierung und Röntgenbilder.

Mit dem Begriff Trainingsdaten werden alle Daten bezeichnet, die den Fitnesszustand des Trainierenden betreffen sowie alle Daten, die mit dem Training in engem Zusammenhang stehen. Dazu gehören unter anderem Alter, Körperfettverteilung, Ausdauerwerte, Messwerte der Herz- und Lungenfunktion unter Belastung, Daten über Kraft und Koordination, Daten über die Erholungsrate nach einer Belastung, Daten über den Blutdruck und den Puls, aber auch Auswertungen vorheriger Trainings, Lasten- und Positionseinstellung von Fitnessgeräten und individuelle Wünsche bezüglich des zu erreichenden Trainingsziels.

Trägt beispielsweise ein Trainer die portable Vorrichtung, so können ihm diese Daten ungefiltert direkt auf einem Display, das beispielsweise in seinem Sichtfeld angeordnet ist, angezeigt werden.

Trägt eine trainierende Person die portable Vorrichtung, so kann ihr alternativ beispielsweise in ihrem Gesichtsfeld eine personalisierte Begrüßungsbotschaft übermittelt werden und/oder ein Teil bzw. eine vereinfachte Darstellung der Gesundheitsdaten und/oder Trainingsdaten angezeigt werden. Ferner kann die portable Vorrichtung zusätzlich auch über wenigstens einen Sensor an der Person, wie z.B. einen Pulsmesser in einem Brustgurt, den Puls der Person direkt abfragen und anzeigen. Diese Daten können dann bei der Verarbeitung oder Auswertung im erfindungsgemäßen Verfahren mit berücksichtigt werden. Alternativ können Messsensoren die Messdaten aber auch an die Datenbank senden, so dass diese Daten von der portablen Vorrichtung mit abgerufen und so im Verfahrensablauf mit berücksichtigt werden können.

In einem weiteren Schritt wird die Bewegungsausführung der identifizierten Person erfasst. Der Begriff der Bewegungsausführung ist im Sinne dieser Anmeldung weit auszulegen. So ist insbesondere auch die Körperhaltung der identifizierten Person während der Bewegung in diesem Begriff mit umfasst. Das Erfassen der Bewegungsausführung kann beispielsweise darin bestehen, dass wenigstens ein Foto oder ein Video der identifizierten Person bei der Ausführung der Übung angefertigt wird. Es können aber auch zusätzlich oder alternativ beispielsweise die Bewegungsamplituden, die Beschleunigung oder die Koordination der Gliedmaßen und Gelenke der identifizierten Person erfasst werden. Hierzu kann insbesondere auch mit Messdaten von Schallwellensensoren und/oder mit Gyrosensoren der portablen Vorrichtung gearbeitet werden. Es ist auch möglich, dass basierend auf den Gesundheitsdaten und den Trainingsdaten eine geeignete Erfassungsmethode durch die portable Vorrichtung bestimmt und zur Erfassung der Bewegungsausführung genutzt wird.

Um die Qualität der Bewegungsausführung zu ermitteln, wird zunächst wenigstens ein Bewegungsmodell für Personen mit entsprechenden Gesundheits- und/oder Trainingsdaten der identifizierten Person zugeordnet. Es kann sich hierbei um ein standardisiertes Modell handeln, das basierend auf den Gesundheitsdaten und/oder den Trainingsdaten der identifizierten Person entsprechend angepasst ist, z.B. durch eine Skalierung. Es kann sich aber auch um ein angelerntes Bewegungsmodell handeln, das beispielsweise durch das Erfassen und Überlagern der Bewegungsausführung verschiedener Trainer und/oder Trainierenden für diese Übungseinheit erstellt wurde und entsprechend an den Gesundheitszustand und/oder den Trainingszustand der identifizierten Person angepasst ist. Es kann sich alternativ auch um ein Bewegungsmodell handeln, das mithilfe der identifizierten Person selbst erstellt wurde, indem sie - z.B. unter Anleitung eines Trainers - bei idealer Bewegungsausführung gefilmt wurde und daraus ein Bewegungsmodell für diese Übung generiert und in der Datenbank hinterlegt worden ist.

Mittels einer Vergleichsanalyse zwischen der erfassten Bewegungsausführung der identifizierten Person mit wenigstens einem in der Datenbank hinterlegten Bewegungsmodell für Personen mit entsprechenden Gesundheitsdaten und/oder Trainingsdaten kann die Qualität der Bewegungsausführung bestimmt werden. Dabei kann ein Gesamtvergleich zwischen Bewegungsmodell und Bewegungsausführung durchgeführt werden, um beispielsweise Abweichungen bei der Bewegungsausführung zu ermitteln. Es kann jedoch zusätzlich oder alternativ auch eine Vergleichsanalyse durchgeführt werden, die auf bestimmte Körperbereiche fokussiert ist, wie z.B. auf die Bewegungsamplitude und die Beschleunigung der Gelenke bei der Bewegungsausführung.

Basierend auf dem Ergebnis der Vergleichsanalyse wird in einem weiteren Schritt des erfindungsgemäßen Verfahrens wenigstens ein optimierter Trainingsparameter generiert. Bei dem Trainingsparameter handelt es sich vorzugsweise um die Art der Bewegungsausführung, die Körperhaltung, die Positionierung auf einem Trainingsgerät oder die Geräteeinstellung. Wird beispielsweise mittels Vergleichsanalyse festgestellt, dass eine Bewegung auf einem Trainingsgerät zu schnell ausgeführt wird, so wird daraus geschlossen, dass die Last am Trainingsgerät zu niedrig eingestellt ist. Die portable Vorrichtung generiert als optimierten Trainingsparameter vorzugsweise die geeignete Last. Diese wird dann auf einem Display der portablen Vorrichtung angezeigt. In einer Variante wird die Last am Trainingsgerät automatisiert nachgeregelt. Wird beispielsweise bei einer Übung eines Trainierenden das Knie des belasteten Beines durchgestreckt, anstatt angewinkelt zu sein, würde dies bei der Vergleichsanalyse festgestellt werden. Als optimierter Trainingsparameter würde dann beispielsweise auf einem Display der portablen Vorrichtung angezeigt werden, dass die Stellung des Knies nicht korrekt ist und wie die Position des Knies unter Berücksichtigung der Gesundheitsdaten und/oder Trainingsdaten der identifizierten Person idealerweise sein sollte. Ferner kann bei einer Vergleichsanalyse beispielsweise festgestellt werden, dass die identifizierte Person bereits für diese Art der Übung zu müde ist. Der optimierte Trainingsparameter würde dann in Form eines Vorschlages generiert werden, nämlich dass die Person eine andere Trainingsübung ausführt, die beispielsweise den Kreislauf wieder ankurbelt.

Da die Leistungsfähigkeit einer Person tagesabhängig ist, sieht das Verfahren bei einer bevorzugten Ausführungsform vor, Leistungsdaten zu quantifizieren und bei der Generierung optimierter Trainingsparameter zu berücksichtigen. So kann die Mimik und/oder die Aussprache der identifizierten Person mittels einer Emotionserkennungseinrichtung bzw. einer Sprachanalyseeinrichtung der portablen Vorrichtung erfasst und analysiert werden. Daraus kann eine tagesabhängige Leistungsfähigkeit z.B. in Form eines Zahlenwertes der identifizierten Person ermittelt werden. Klagt beispielsweise die identifizierte Person über Rückenschmerzen, so können aus dieser Information Variationsmöglichkeiten der Bewegungsausführung generiert und der trainierenden Person angezeigt werden. Wird beispielsweise festgestellt, dass die tagesabhängige Leistungsfähigkeit vermindert ist, so kann beispielsweise die Last des Gerätes automatisiert reduziert werden.

Um in einer bevorzugten Variante die Qualität der Vergleichsanalyse zu verbessern, ist es wichtig, dass die Person grundsätzlich weiß, wie ein optimaler Bewegungsablauf einer Übung auf einem Trainingsgerät auszusehen hat. Daher ist bei einer bevorzugten Ausführungsform vorgesehen, dass wenigstens ein Trainingsgerät automatisiert erkannt wird und der identifizierten Person ein Bewegungsablauf einer Übung auf dem Trainingsgerät und/oder individualisierte Einstellungen für das Trainingsgerät auf einem Display der portablen Vorrichtung angezeigt werden. Dies kann beispielsweise in Form eines Trainingsvideos erfolgen.

Häufig werden Knochen- und Gelenkserkrankungen zu spät diagnostiziert, so dass solche Krankheiten bereits weit fortgeschritten sind, bis sie erkannt werden. Eine Linderung der Beschwerden ist dann meistens nur mit erheblichem Aufwand möglich. Daher ist es erstrebenswert, solche Knochen- und Gelenkserkrankungen so früh wie möglich zu diagnostizieren, am besten zu einem Zeitpunkt, wenn die erkrankte Person noch keine Verschlechterung ihrer Lebensqualität verspürt. Um diese frühzeitige Diagnose zu ermöglichen, sieht das Verfahren bei einer bevorzugten Ausführungsform vor, dass zusätzlich eine Vergleichsanalyse der Körperhaltung und/oder der Gelenkbewegung der identifizierten Person mit wenigstens einem in der Datenbank hinterlegten Köperhaltungsmodell bzw. Gelenkbewegungsmodell für Personen mit entsprechenden Gesundheits- und/oder Trainingsdaten durchgeführt wird. Basierend auf dieser durchgeführten Vergleichsanalyse können dann Knochen- und Gelenkserkrankungen diagnostiziert oder Wahrscheinlichkeiten für das Auftreten solcher Krankheiten ermittelt werden.

Das erfindungsgemäße Verfahren soll auch dazu dienen, die Arbeit eines Trainers zu erleichtern. Demnach ist bei einer bevorzugten Ausführungsform vorgesehen, dass Daten aus der Datenbank möglichst einfach durch Gesten abgerufen und editiert werden können. Dies kann sogar während der Durchführung des Verfahrens erfolgen. So kann der Trainer bei Bedarf - z.B. bei zu hoher gemessener Herzfrequenz oder Blutdruck - mit einer Handgeste Erinnerungsmarker setzen, um Einzelheiten nach dem Training mit der identifizierten Person zu besprechen. Außerdem können Trainingspläne durch Gestik angepasst oder Trainingsdaten modifiziert werden.

Ferner sieht das Verfahren bei einer bevorzugten Ausführungsform vor, dass die Positionierung von Sensoren und/oder Elektroden und/oder der Wartungszustand und/oder der Gerätestatus eines Trainingsgerätes durch die portable Vorrichtung bestimmt wird und im Falle von Abweichungen zu den vorgegebenen Werten aus einer Datenbank entsprechende Verbesserungsmöglichkeiten ausgewählt und optisch und/oder akustisch ausgegeben werden.

Dies sorgt für eine Entlastung des Trainers, der sich voll und ganz auf die Bewegungsausführungen der Trainierenden konzentrieren kann.

Bei einer falschen Bewegungsausführung - insbesondere bei ruckartigen Bewegungen - werden Muskeln, Faszien und Bänder enorm strapaziert. Meist reicht dann schon eine falsche Bewegung aus und die Muskeln verhärten sich. Um hier noch während des falschen Bewegungsablaufs eingreifen zu können, ist bei einer bevorzugten Ausführungsform des erfindungsgemäßen Verfahrens vorgesehen, dass die portable Vorrichtung ein visuelles, akustisches und/oder vibronisches Warnsignal abgibt, falls bei der Vergleichsanalyse ein voreingestellter oder gewählter Grenzwert unterschritten oder überschritten wird.

Damit beispielsweise die Kommunikation zwischen einem Trainer und der identifizierten Person für den Trainer leichter wird, ist bei einer bevorzugten Ausführungsform vorgesehen, dass basierend auf der analysierten Mimik und/oder dem Gesprochenen der identifizierten Person vorformulierte Textbausteine und/oder thematische Hintergrundinformationen aus der Datenbank ausgewählt und von der portablen Vorrichtung visuell und/oder akustisch ausgegeben werden. Diese Textbausteine und/oder thematische Hintergrundinformationen, wie z.B. die gespeicherte Familienhistorie der identifizierten Person, kann der Trainer dann in das Gespräch einbauen.

Berichtet die identifizierte Person beispielsweise von ihren Rückenproblemen, so wird der ausgesprochene Satz analysiert und aus der Datenbank z.B. ein entsprechendes Leistungsangebot für Rückenprobleme des Studios aufgerufen. Der Trainer kann dieses dann der identifizierten Person vorstellen.

Weitere Hintergrundinformationen sind beispielsweise die Anzeige vorheriger Bestellungen der identifizierten Person, Kosten von Leistungen des Studios und durchschnittliche Lieferzeiten von Waren.

Gegenstand der vorliegenden Erfindung ist ferner ein System zur Erfassung, Analyse und Optimierung von Bewegungsausführungen im Sportbereich und Rehabereich. Das System umfasst wenigstens eine Datenbank mit hinterlegten Fotos und/oder biometrischen Daten sowie dazu zugeordneten Gesundheits- und/oder Trainingsdaten von Personen. Ferner umfasst das System wenigstens eine portable Vorrichtung, die folgende Komponenten umfasst:
- eine Gesichtserkennungseinrichtung und/oder eine Erkennungseinrichtung für biometrische Merkmale zur Identifikation einer Person im Training;
- wenigstens eine Verarbeitungseinrichtung, die auf die Datenbank zugreifen kann, um in der Datenbank hinterlegte Gesundheits- und/oder Trainingsdaten der identifizierten Person abzurufen;
- wenigstens eine Erfassungseinrichtung, um die Bewegungsausführung der identifizierten Person zu erfassen;
wobei die Verarbeitungseinrichtung der identifizierten Person wenigstens ein Bewegungsmodell der Person mit entsprechenden Gesundheitsdaten und/oder Trainingsdaten zuordnet und eine Vergleichsanalyse zwischen der erfassten Bewegungsausführung und dem wenigstens einen zugeordneten Bewegungsmodell durchführt, so dass die Verarbeitungseinrichtung aus den abgeglichenen Daten wenigstens einen optimierten Trainingsparameter generiert, der für das Training verwendbar ist.

Bei einer bevorzugten Ausführungsform handelt es sich bei der portablen Vorrichtung um eine Digital-Reality (DR) Brille, insbesondere um eine Virtual-Reality (VR) Brille, eine Augmented-Reality (AR) Brille oder eine Mixed-Reality (MR) Brille. Mit einer solchen Brille kann die Benutzererfahrung erweitert werden. So können z.B. virtuelle Sportbereiche wie z.B. ein Tennisfeld oder Equipment dargestellt werden. Es können optimierte Laufwege angezeigt werden, um beispielsweise einem Benutzer einer solchen Brille unter Berücksichtigung der Auslastung des Fitnessstudios eine Reihenfolge für die Gerätenutzung vorzugeben, in der er sein Training ausführen sollte. Ferner können mit Hilfe einer solchen Brille beispielsweise Patienten- und Kundendaten in einem 3D-Modell dargestellt oder Trainingsflächen virtuell geplant, Produktkataloge dargestellt oder Werbung für den Benutzer eingeblendet werden.

Eine Ausführungsvariante der vorliegenden Erfindung wird exemplarisch anhand der beigefügten Zeichnung näher erläutert:

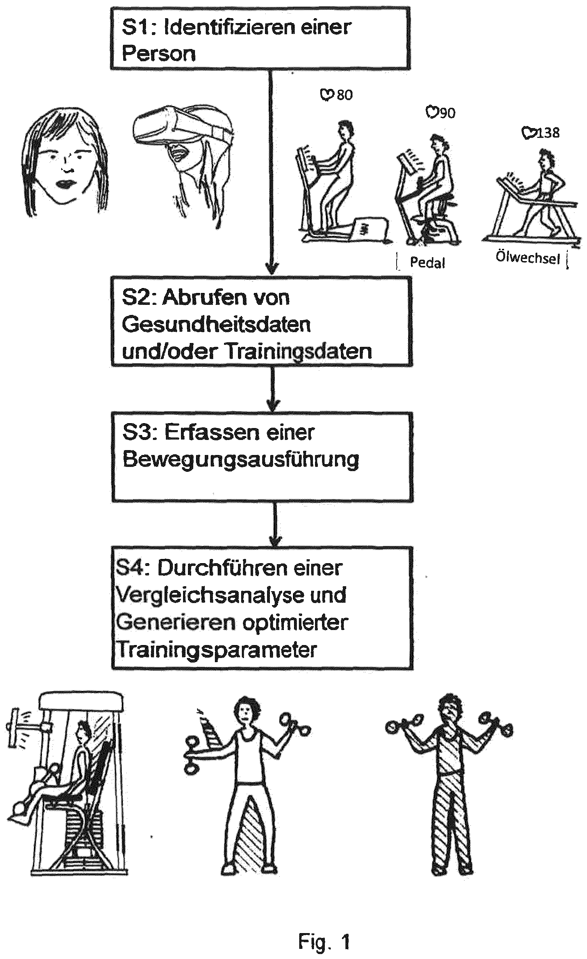

Figur 1 zeigt in einem Flussdiagramm den generellen Ablauf des Verfahrens. In der gezeigten Ausführungsform trägt eine Trainerin eine Digital-Reality (DR) Brille, z.B. eine Mixed-Reality Brille. Die Trainerin kann sich mit Hilfe dieser Brille in einem Raum umsehen. Dabei sieht sie sowohl den tatsächlichen Raum mit Personen, die beispielsweise auf Trainingsgeräten trainieren, als auch eingeblendete Informationen, beispielsweise über den Wartungszustand eines Trainingsgerätes. Schaut sich die Trainerin das Gesicht einer zu identifizierenden Person an, so wird das Gesicht der zu identifizierenden Person von einer Gesichtserkennungseinrichtung der DR Brille erfasst und mit Hilfe eines Abgleichs von Gesichtsmerkmalen mit Merkmalen, die in einer Datenbank hinterlegt sind, identifiziert. Hierzu greift die Brille über ein drahtgebundenes oder drahtloses lokales Datennetz auf diese Datenbank zu. Alternativ kann die Datenbank in der DR Brille integriert sein. Eine Identifizierung der Person kann auch über biometrische Merkmale, wie z.B. mittels eines Irisscans erfolgen. Diese Identifikationsvariante ist besonders interessant, falls die zu identifizierende Person die Brille selbst aufgesetzt hat (nicht dargestellt).

In einem zweiten Schritt werden über die Brille die Gesundheits- und/oder Trainingsdaten der identifizierten Person aus der Datenbank abgerufen. Diese Daten werden in einem Sichtfeld der DR Brille dargestellt. Mit Hilfe von Handgesten kann die Trainerin diese Daten editieren, abspeichern und an die Datenbank übertragen. Zusätzlich kann die identifizierte Person Messsensoren wie z.B. einen Pulsmesser tragen. Diese Daten können von der DR Brille abgerufen und der Trainerin unmittelbar angezeigt werden. Ferner können diese Messdaten auch an die Datenbank über das drahtlose oder lokale Datennetz gesendet werden und auf diese Weise bei der Ausführung des Verfahrens von der DR Brille abgerufen werden.

In einem dritten Schritt wird über die Erfassungseinrichtung der DR Brille die Bewegungsausführung der identifizierten Person erfasst. Das Erfassen der Bewegungsausführung kann beispielsweise darin bestehen, dass ein Foto oder ein Video der identifizierten Person bei der Ausführung der Übung erstellt wird. Zusätzlich oder alternativ können beispielsweise die Bewegungsamplituden, die Beschleunigung oder die Koordination der Gliedmaßen und Gelenke der identifizierten Person erfasst werden.

Die erfasste Bewegungsausführung wird analysiert. Dies erfolgt im Rahmen einer Vergleichsanalyse mit wenigstens einem Bewegungsmodell, welches der identifizierten Person entsprechend ihren Gesundheits- und/oder Trainingsdaten zugeordnet wird. Dabei kann ein Gesamtvergleich zwischen Bewegungsmodell und Bewegungsausführung durchgeführt werden, um Abweichungen zu ermitteln. Die Analyse kann sich aber auch auf die Bewegung einzelner Körperbereiche beschränken.

Im gezeigten Ausführungsbeispiel ergibt die Vergleichsanalyse, dass sich der Oberkörper der Person auf dem Beinstrecker während der Ausführung der Übungseinheit von der Rückenlehne des Beinstreckers abhebt (linkes Bild). Diese Haltung ist nicht gewünscht, da bei der großen Last an diesem Trainingsgerät Schädigungen am Rücken der identifizierten Person entstehen können. Diese von einem Trainer leicht zu übersehende Fehlhaltung wird der Trainerin unmittelbar auf dem Display der DR Brille angezeigt, sobald sie in Richtung der Person auf dem Beinstrecker blickt. Aufgrund der starken Abweichung der Bewegungsausführung der Person auf dem Beinstrecker im Vergleich zum zugrundeliegenden Bewegungsmodell für diese Person wird zusätzlich ein visuelles, akustisches und/oder vibronisches Warnsignal von der DR Brille abgegeben.

Trägt die trainierende Person die Brille (nicht gezeigt), so würde die Brille ihr im dargestellten exemplarischen Fall anzeigen, dass sie eine Fehlhaltung eingenommen hat und zusätzlich ein Warnsignal abgeben, z.B. indem die Brille vibriert oder farbig leuchtet. Ergänzend könnte beispielsweise ein Video in die Brille eingespielt werden, das die korrekte Bewegungsausführung auf diesem Trainingsgerät erklärt oder eine virtuelle Animation der korrekten Bewegungsausführung darstellt.

Im rechten Beispiel ist eine Person gezeigt, die eine Hantelübung ausführt. Jedoch ist deren Körperhaltung bei dieser Übung nicht optimal. Blickt die Trainerin mit der aufgesetzten DR Brille auf diese Person, so wird diese zunächst identifiziert, die Gesundheitsdaten und/oder Trainingsdaten von einer Datenbank abgerufen, die Bewegungsausführung erfasst, ihr ein geeignetes Bewegungsmodell zugeordnet und eine Vergleichsanalyse zwischen Bewegungsmodell und erfasster Bewegungsausführung durchgeführt. Die Vergleichsanalyse ergibt, dass der rechte Arm der identifizierten Person zu niedrig ist und ihre Beine zu weit auseinander gespreizt sind. Die Brille macht auf diese fehlerhafte Körperhaltung aufmerksam und ermittelt als optimierten Trainingsparameter die korrekte Körperhaltung und zeigt diese an. Die Trainerin informiert daraufhin die identifizierte Person über ihre fehlerhafte Körperhaltung. Während dieses Informationsgespräches analysiert eine Sprachanalyseeinrichtung der DR Brille das Gesprochene der identifizierten Person. Im vorliegenden Beispiel wird anhand der Analyse festgestellt, dass die Person mit den Hanteln Schmerzen im Rücken hat und daher das Hanteltraining nicht sorgfältig ausführt. Außerdem ergibt die Analyse, dass die Person zu unmotiviert ist, um das Hanteltraining vernünftig fortzusetzen. Der Trainerin werden daraufhin ausgewählte Textbausteine aus der Datenbank angezeigt, die der Motivation der trainierenden Person für das Hanteltraining dienen sollen. Diese Textbausteine kann die Trainerin der trainierenden Person vortragen, um sie für das Hanteltraining zu motivieren. Ferner werden der Trainerin auf dem Display der DR Brille Übungen für Rückenprobleme angezeigt, welche sie der identifizierten Person vorstellen kann. Ferner kann ihr auch ein Leistungsangebot des Studios für entsprechende Kurse auf dem Display der VR Brille eingespielt werden.

Das oben beschriebene Beispiel schränkt den Erfindungsgedanken der vorliegenden Erfindung nicht ein. Es versteht sich, dass anstelle der VR Brille auch eine alternative portable Vorrichtung wie beispielsweise ein Tablet oder ein Smartphone den Erfindungsgedanken umsetzen kann. Ferner ist beim Erfindungsgedanken inbegriffen, dass ein Trainer oder ein Trainierender oder beide die portable Vorrichtung verwenden. Auch ist es möglich, dass mehrere portable Vorrichtungen miteinander kommunizieren und Daten austauschen. Auch können die portablen Vorrichtungen mit weiteren externen Geräten wie z.B. einer Kamera an einem Trainingsgerät oder einem Drucker kommunizieren. Ferner kann das Verfahren bzw. das System auch von zu Hause aus, in der freien Natur oder an öffentlichen Plätzen angewendet bzw. verwendet werden. Es versteht sich, dass die ermittelten optimierten Trainingsparameter an die Datenbank übermittelt und dort gespeichert werden und so einer wiederholten Ausführung des Verfahrens zur Verfügung stehen.

## Patentansprüche

1. Verfahren zur Erfassung, Analyse und Optimierung von Bewegungsausführungen im Sportbereich und Rehabereich, umfassend die folgende Schritte:
- Identifizieren einer Person durch Abgleich mit wenigstens einem in einer Datenbank hinterlegten Foto und/oder wenigsten einem biometrischen Merkmal mittels einer auf die Datenbank zugreifenden Gesichtserkennungseinrichtung und/oder einer Erkennungseinrichtung für biometrische Merkmale einer portablen Vorrichtung;
- Abrufen von Gesundheitsdaten und/oder Trainingsdaten zur identifizierten Person aus der Datenbank;
- Erfassen wenigstens einer Bewegungsausführung der identifizierten Person;
- Zuordnen und Durchführen einer Vergleichsanalyse der erfassten Bewegungsausführung der identifizierten Person mit wenigstens einem in der Datenbank hinterlegten Bewegungsmodell für Personen mit entsprechenden Gesundheitsdaten und/oder Trainingsdaten;
- Generieren wenigstens eines optimierten Trainingsparameters aus der durchgeführten Vergleichsanalyse und
- Verwenden des wenigstens einen optimierten Trainingsparameters zur Optimierung der Bewegungsausführung.

2. Verfahren nach Anspruch 1, **dadurch gekennzeichnet, dass** die Mimik und/oder das Gesprochene der identifizierten Person mittels einer Emotionserkennungseinrichtung und/oder einer Sprachanalyseeinrichtung der portablen Vorrichtung erfasst und analysiert wird, um eine tagesabhängige Leistungsfähigkeit der Person im Training zu ermitteln, welche bei der Generierung der optimierten Trainingsparameter berücksichtigt wird.

3. Verfahren nach einem der vorhergehenden Ansprüche, **dadurch gekennzeichnet, dass** wenigstens ein Trainingsgerät automatisiert erkannt wird und der identifizierten Person ein Bewegungsablauf einer Übung auf dem Trainingsgerät und/oder individualisierte Einstellungen für das Trainingsgerät auf einem Display der portablen Vorrichtung angezeigt werden.

4. Verfahren nach einem der vorhergehenden Ansprüche, **gekennzeichnet durch** die folgenden zusätzlichen Schritte:
- Durchführen einer Vergleichsanalyse der Körperhaltung und/oder der Gelenkbewegungen der identifizierten Person mit wenigstens einem in der Datenbank hinterlegten Körperhaltungsmodell oder Gelenkbewegungsmodell für Personen mit entsprechenden Gesundheitsdaten und/oder Trainingsdaten, und
- Prognose der Wahrscheinlichkeit einer Knochen- und Gelenkserkrankung.

5. Verfahren nach einem der vorhergehenden Ansprüche, **dadurch gekennzeichnet, dass** es sich bei den Trainingsparametern um die Art der Bewegungsausführung und/oder die Körperhaltung und/oder die Positionierung auf einem Trainingsgerät und/oder die Geräteeinstellung handelt.

6. Verfahren nach einem der vorhergehenden Ansprüche, **dadurch gekennzeichnet, dass** Daten aus der Datenbank durch Gesten abgerufen und editiert werden können.

7. Verfahren nach einem der vorhergehenden Ansprüche, **dadurch gekennzeichnet, dass** die Positionierung von Sensoren und/oder Elektroden und/oder der Wartungszustand und/oder der Gerätestatus eines Trainingsgerätes durch die portable Vorrichtung bestimmt wird und im Falle von Abweichungen zu vorgegebenen Werten aus einer Datenbank entsprechende Verbesserungsmöglichkeiten ausgewählt und optisch und/oder akustisch ausgegeben werden.

8. Verfahren nach einem der vorhergehenden Ansprüche, **dadurch gekennzeichnet, dass** die portable Vorrichtung ein visuelles, akustisches und/oder vibronisches Warnsignal abgibt, falls bei der Vergleichsanalyse ein voreingestellter oder gewählter Grenzwert unterschritten oder überschritten wird.

9. Verfahren nach einem der Ansprüche 2 bis 8, **dadurch gekennzeichnet, dass** basierend auf der analysierten Mimik und/oder dem Gesprochenen der identifizierten Person vorformulierte Textbausteine und/oder thematische Hintergrundinformationen aus der Datenbank ausgewählt und von der portablen Vorrichtung visuell und/oder akustisch ausgegeben werden.

10. System zur Erfassung, Analyse und Optimierung von Bewegungsausführungen im Sportbereich und Rehabereich, umfassend
- wenigstens eine Datenbank mit wenigstens einem hinterlegten Foto und/oder einem biometrischen Merkmal sowie dazu zugeordneten Gesundheitsdaten und/oder Trainingsdaten von Personen;
- wenigstens eine portable Vorrichtung, die folgende Komponenten umfasst:
- eine Gesichtserkennungseinrichtung und/oder eine Erkennungseinrichtung für biometrische Merkmale zur Identifikation einer Person im Training,
- wenigstens eine Verarbeitungseinrichtung, die auf die Datenbank zugreifen kann, um in der Datenbank hinterlegte Gesundheitsdaten und/oder Trainingsdaten der identifizierten Person abzurufen;
- wenigstens eine Erfassungseinrichtung, um eine Bewegungsausführung der identifizierten Person zu erfassen;
wobei die Verarbeitungseinrichtung der identifizierten Person wenigstens ein Bewegungsmodell für Personen mit entsprechenden Gesundheitsdaten und/oder Trainingsdaten zuordnet und eine Vergleichsanalyse zwischen der erfassten Bewegungsausführung und dem wenigstens einen zugeordneten Bewegungsmodell durchführt, so dass die Verarbeitungseinrichtung aus den abgeglichenen Daten wenigstens einen optimierten Trainingsparameter generiert, der zur Optimierung der Bewegungsausführung verwendbar ist.

11. System nach Anspruch 10, **dadurch gekennzeichnet, dass** es sich bei der portablen Vorrichtung um eine Digital-Reality (DR) Brille, insbesondere um eine Virtual-Reality (VR) Brille, eine Augmented-Reality (AR) Brille oder eine Mixed-Reality (MR) Brille handelt, mit der insbesondere virtuelle Trainingsumgebungen und/oder Trainingsequipment und/oder optimierte Routen darstellbar sind.
